Europäisches Patentamt

European Patent Office

Office européen des brevets

(11) Publication number: **0 201 553 B1**

# EUROPEAN PATENT SPECIFICATION

(45) Date of publication of patent specification: **24.06.92**  (51) Int. Cl.⁵: **A61L 2/12**, A61L 2/24

(21) Application number: **85905637.6**

(22) Date of filing: **08.11.85**

(86) International application number:
**PCT/GB85/00509**

(87) International publication number:
**WO 86/02842 (22.05.86 86/11)**

(54) **STERILISING PROCEDURES AND EQUIPMENT.**

(30) Priority: **10.11.84 GB 8428470**

(43) Date of publication of application:
**20.11.86 Bulletin  86/47**

(45) Publication of the grant of the patent:
**24.06.92 Bulletin  92/26**

(84) Designated Contracting States:
**AT BE CH DE FR GB IT LI LU NL SE**

(56) References cited:
**FR-A- 2 547 732**
**US-A- 3 676 058**
**US-A- 3 753 651**

**American Journal of Hospital Pharmacy, vol.
29, August 1972, Philadelphia College of
Pharmacy (Philadelphia,US), R.M.G. Boucher:
"Advances in sterilization techniques",
pages 661-672, see pages 668 and 671**

(73) Proprietor: **University of Wales College of
Medicine
Heath Park
Cardiff CF4 4XN(GB)**

(72) Inventor: **MATTHEWS, Ian, Price
6 Boleyn Walk Penylan
Cardiff(GB)**
Inventor: **SAMUEL, Alan, Herbert 22 Foreland
Road
Whitchurch
Cardiff CF4 7AR(GB)**

(74) Representative: **Lainé, Simon James et al
Wynne-Jones, Lainé & James Morgan Ar-
cade Chambers 33, St.Mary Street
Cardiff CF1 2AB(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the sterilising of articles to kill or destroy bacteria or other unwanted or toxic bodies and/or the desorption of gas or vapour from articles. The invention is of particular utility in the sterilising of equipment used for medical or surgical purposes, but may have other applications, for example in industry.

It is of course well-known to sterilise metal surgical instruments by steam and it has also been common practice to sterilise various articles in sterilising liquids. High temperature steam sterilising is simple and effective for certain metal objects, but has limitations and disadvantages, for example it is inefficient in sterilising any object within a sealed package, and the high temperatures involved may cause degradation of plastic articles. The use of sterilising liquids also has limitations, for example air bubbles may be trapped within the object thus preventing full effectiveness, and it may be difficult or impossible to remove all trace of the liquid afterwards.

It has also been common practice to sterilize medical or surgical equipment by means of a toxic gas and many existing sterilisers in hospital use, for example,involve contacting the articles with Ethylene Oxide in a sealed sterilising chamber. Ethylene Oxide is an extremely effective bactericide, and is effective not only for metal objects such as surgical implements, but also relatively delicate instruments and equipment, including synthetic plastics. The gas is effective at medium or low temperatures and thus the effects of high temperatures on degrading materials can be avoided. The gas penetrates or permeates plastic wrappings and can thus be used to sterilise pre-packaged objects.

Ethylene Oxide is, however, extremely toxic to human beings and after its use for sterilizing it is important that steps be taken to remove all traces, as far as possible. To this end it is known to flush out the interior of the sterilizing chamber or autoclave with fresh air after the sterilizing gas has been extracted. It has also been proposed to "pulse" or vary the pressure of the flushing air during the flushing operation.

One of the problems involved is that the Ethylene Oxide is absorbed into a number of typical plastic materials which may need to be sterilized, and to "desorb" the gas from the plastics appears to be extremely difficult. Even after the sterilising chamber has been flushed the desorption of the gas from the objects may result in contaminating the atmosphere of the laboratory or other workroom to a concentration of, say, 5 ppm or more which is an unacceptable health hazard.

The invention is based on the discovery that microwave energy has important and surprising advantages in the sterilizing field. As will be explained more fully it has a valuable effect in aiding the desorbtion of a gas such as ethylene oxide, and it also has a valuable synergistic effect in the actual sterilising process when used in conjunction with ethylene oxide. More particularly the invention is based in part on the discovery that microwave energy exerts a non-thermal as well as a thermal effect upon materials, and the synergistic use of microwave radiation in conjunction with ethylene oxide gas for sterilization is far more effective than use of either agent alone. This is believed to follow from two allied but different reasons.

Non-thermal effects result directly from the interaction of the electromagnetic field with molecules. When polar molecules are subjected to a strong alternating electromagnetic field their tendency to orientate with the applied field causes a degree of molecular disturbance. Thermal effects, on the other hand, are a consequence of "dielectric loss" in which part of the applied field voltage is resolved as Joule heating due to the inability of the molecular dipole to align with the field with absolute fidelity, i.e. the molecular orientations are slightly out of phase with the applied field due to hindered movement. It has been found that microwaves exert a biocidal effect distinct from that of thermal energy, and it is believed that this may result from their effect on metabolic chemistry, possibly by causing alternations in cell membrane permeability, or by altering the pH (acidity) of the cellular environment, or by modifying weak chemical bonds between cellular macro molecules. Importantly also it is believed that a very advantageous non-thermal biocidal effect of microwaves lies in the degree of absorption of microwave energy by key molecules such as deoxyribonucleic acid (DNA). For these reasons it is believed that microwave irradiated micro-organisms are less viable and more susceptible to destruction by ethylene oxide gas. A mode of action involving direct effect upon cellular DNA particularly favours the synergistic use with ethylene oxide gas, which also exerts a direct effect upon DNA.

The biocidal efficacy of ethylene oxide is a consequence of its electrophilic and lipophillic nature and the reactivity of the epoxide ring. It combines very readily with nucleophilic centres on nucleic acids and proteins. The mechanism of DNA alkylation by ethylene oxide is principally at the site of the $N_7$ - atom of the base guanine leading to $N_7$ - (2-hydroxyethyl) guanine. It is believed that the interaction of the applied microwave electric field with the bonding electrons in molecules of ethylene oxide may induce further significant polarization of bonds, thus enhancing the electrophilic reactivity of the molecule.

For sterilization purposes ethylene oxide gas has sometimes been mixed with freon or carbon dioxide in the proportion of for example 12% E.O.and 88% freon or $CO_2$. In performing the present invention it is possible to use the gas to transfer heat from the material to be sterilized by recycling and compressing the gas (as in a conventional refrigeration process). This may permit a much greater input of microwave energy into the materials thereby enhancing the non-thermal effects whilst maintaining materials at an acceptable temperature.

US-A-3676058 teaches a method in which articles to be sterilised are individually packed and sealed in gas impermeable containers and then irradiated whilst the exteriors of the containers are cooled by a coolant gas.

US-A-3753651 teaches a method in which the articles to be sterilised are sealed in a single sealed gas impermeable container which is then inserted in a microwave chamber to be irradiated.

The American Journal of Hospital Pharmacy, Volume 29, pp 661-672 mentions the use of microwave energy in conjunction with ethylene oxide.

Accordingly it is an object of the invention to provide an improved procedure and equipment for sterilising and/or "desorbing" objects and reducing or limiting some of the problems outlined above.

Broadly stated from one aspect the invention consists in sterilising apparatus comprising a chamber having an access opening and a closure thereof, fluid-tight sealing means for the closure, means for introducing a sterilising gas or vapour into the chamber and for extracting the gas or vapour when required, means for generating or radiating high frequency electro-magnetic radiation in or into the chamber, means to limit or prevent unwanted escape or discharge of the radiated energy, and means for creating a fluid-tight seal with the closure.

Preferably the closure seal is also provided with means to restrict or prevent escape of electromagnetic radiation and the apparatus also preferably includes a movable support for objects to be sterilized within the chamber, and drive means operable from outside the chamber for actuating the support. The drive means may include a drive coupling between the interior and exterior of the chamber, the coupling being arranged to maintain the positive fluid and radiation seals.

According to another preferred feature of the invention the apparatus includes means to locate a sealed container for the gas or liquid within the chamber and means to discharge the fluid from the container within the chamber, the actuating means being operable from outside the chamber without disturbing the gas or radiation seals.

The radiation generator may be a microwave irradiation system arranged to create or introduce microwave radiation into the chamber through a sealed wall, "transparent" to the microwave radiation,and the generator may also include means for varying the level of radiation.

Preferably the apparatus includes means for varying or controlling the pressure within the chamber, e.g. above or below atmospheric.

From another aspect the invention consists in a method of sterilising an article in which the article is placed in a sealed chamber, sterilising gas or vapour is introduced into the chamber and later withdrawn, high frequency electro-magnetic radiation is created or introduced into the chamber to cause deposition of energy into the article and/or any adsorbed gas or vapour, and hence desorption thereof, and the article is then withdrawn from the chamber.

The article is conveniently introduced into and withdrawn from the chamber through a closure which includes a fluid-tight seal and a radiation seal.

The gas is conveniently Ethylene Oxide, which is extremely toxic to human beings and preferably the sterilising gas, or liquid, is initially contained in a sealed capsule or container placed within the chamber, and the gas is released from the container by an actuator operated externally of the chamber.

Conventional thermometers are not effective in a microwave field and according to another preferred feature of the invention the temperature within the chamber, or of the article, is sensed from outside the chamber by an infra-red pyrometer or other remote thermal sensor unaffected by the radiation.

The microwave radiation will normally be created in the chamber after the sterilising gas or vapour has been withdrawn so as to encourage desorption of the gas or vapour into the surrounding air or other atmosphere. It may, however, in some cases be of advantage to introduce or create the radiation in the chamber while the sterilising gas or vapour is within the chamber and this may assist the sterilising process.

In any case the article to be sterilised is preferably moved or rotated within the chamber during exposure to the gas or vapour and/or the radiation by means of a motivating system actuated externally.

The invention may also be applied as an additional feature to existing sterilising equipment and procedures. Thus from another aspect the invention also consists in gas desorbing apparatus for use in conjunction with gas sterilising apparatus, comprising a fluid tight chamber having an access opening and a closure and fluid tight sealing means for the closure, means for introducing a cleansing or flushing gas or vapour such as air into the chamber and for extracting the said gas or vapour, means for generating or radiating high-frequency electromagnetic radiation in or into the chamber, and means to limit or prevent unwanted escape or discharge of the radiated energy.

Likewise the invention also consists in a method of desorbing gas or vapour absorbed into an article for sterilisation, in which the article is placed in a sealed desorbing chamber, high-frequency electromagnetic radiation is created in or introduced into the chamber to cause deposition of energy into the article and/or any absorbed gas or vapour, and hence desorption of the gas or vapour, and the article is then removed from the chamber.

According to a preferred feature of the invention the apparatus may include automatic or semi-automatic systems for controlling the cycle, comprising one or more sensors for detecting selected parameters of the operating conditions such as chamber pressure, or temperature, radiation level, concentration of gas or vapour, and physical state of actuating elements, together with a programmed controller, and automatic control function elements actuated by the controller for performing selected steps in the cycle.

In a further aspect the invention provides apparatus for sensing or measuring a gas vapour or constituent, comprising a chamber into which the gas vapour or constituent is introduced, means for generating or introducing microwave radiation in the chamber and means for tuning the resonant cavity afforded by the chamber, wherein the tuning means comprises a piezoelectric positioning element connected to a movable member or wall in the chamber.

In another aspect the invention provides apparatus for sensing or measuring a gas vapour or constituent, comprising a chamber into which the gas vapour or constituent is introduced, means for generating or introducing microwave radiation in the chamber and means for tuning the resonant cavity afforded by the chamber wherein multiple sequential scanning modulation is applied to at least one of the resonant frequency of the resonant cavity and the frequency of the microwave radiation.

The invention may be performed in various ways and one specific embodiment will now be described by way of example with reference to the accompanying drawings, in which:-

Figure 1 is a diagrammatic side elevation of a gas sterilising apparatus according to the invention,

Figure 2 is a diagrammatic plan view of the apparatus,

Figure 3 is a diagrammatic side elevation on an enlarged scale illustrating the drive mechanism and coupling for rotating the internal support basket,

Figure 4 is a diagrammatic view illustrating two components of the drive coupling,

Figure 5 is a diagrammatic side elevation on an enlarged scale illustrating the sealed internal gas release device,

Figure 6 is a diagrammatic end view illustrating the housing for the capsule puncturing needle,

Figure 7 is a diagram illustrating a microwave spectrometer for detecting Ethylene Oxide in the equipment,

Figure 8 is a diagram illustrating components of a microwave spectrometer system for use in performing the invention, and

Figure 9 is a diagram illustrating a general automatic control system for a sterilising/desorbing cycle.

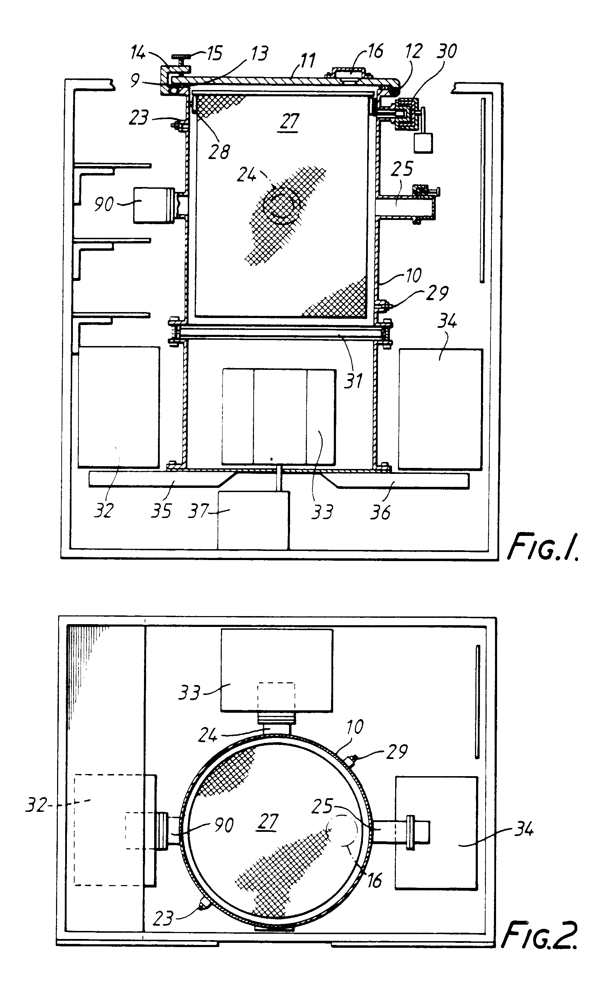

Referring first to the essential elements of the apparatus illustrated in Figures 1 and 2, the equipment comprises a sterilising chamber 10 having a stainless steel cylindrical side wall designed to withstand internal pressures, for example of up to five atmospheres, or a partial vacuum. The top of the chamber is closed by a circular lid or cap 11, which is hinged to the side wall at 12 and provided with a circumferential O-ring or other fluid seal 13, and a metallic knitmesh microwave seal 9. The lid can be held closed by a hinged clamp 14 provided with a tightening screw 15. The lid may also have a rupturable pressure relief disc valve 16, to prevent build up of excessive internal pressures.

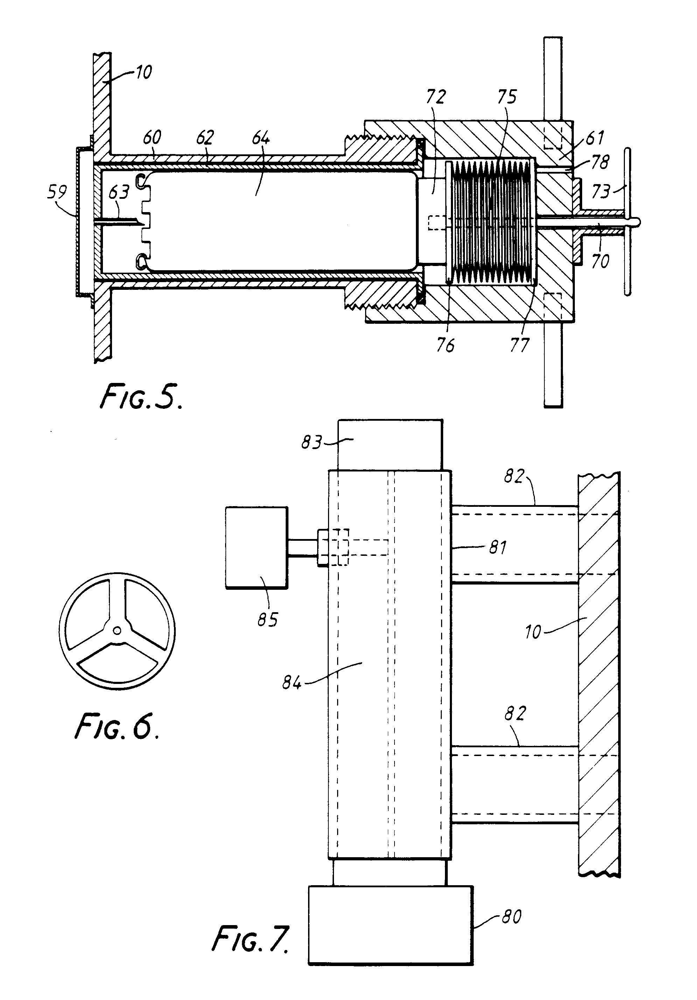

On the side of the chamber 10 is an air entry 23 which may be connected to a source of supply for a controlled selected gas, or atmosphere, as required. The side wall of the chamber is also provided with another port 29 intended to act as an air outlet, and this may be connected to a special vent duct with a filter, or to atmosphere, as required. The filter conveniently includes a mass of high molecular weight polymeric material designed to adsorb Ethylene Oxide, from which the gas can later be reclaimed for disposal or recycling. The wall of the chamber is also provided with a microwave spectrometer 24, as illustrated in Figure 2, and at another part of the side wall is a special sealed injector unit 25 for admitting controlled quantities of Ethylene Oxide when required. This is illustrated in greater detail in Figures 5 and 6.

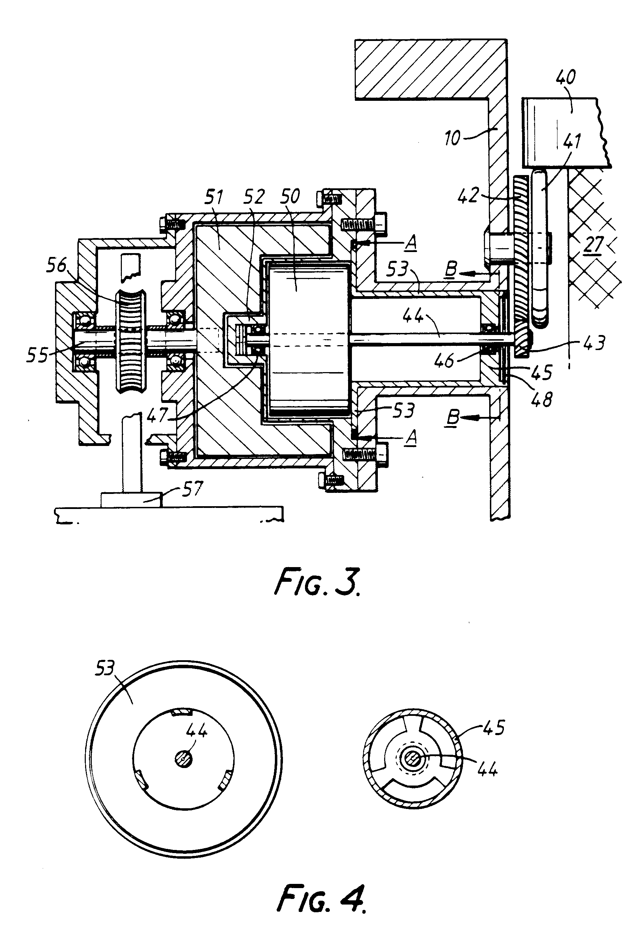

Within the chamber is a rotary basket 27 for equipment to be sterilised, the basket being supported by bearings 28 mounted on the side wall of a chamber and a special drive system 30 for causing rotation of the basket is illustrated in detail in Figures 3 and 4. The bottom wall 31 of the chamber is formed of a material such as Polypropylene which is capable of transmitting electromagnetic radiation and acts as a microwave window, and below the chamber are located three microwave generators 32,33,34 associated with launching "horns" 35,36 and a "mode stirrer" 38 driven by a motor 37. Means are provided to vary the output of the generator 33 and to augment this as required by one or both of the generators 32,34 so as to provide for variation over all three ranges.

The drive mechanism for the basket 27, as illustrated in Figures 3 and 4, is arranged to provide for effective drive coupling without disturbing the microwave and fluid seals. As illustrated, the basket 27 has an upper rim 40 which is supported on the free bearing rollers 28 and at one point on a driven roller 41 within the chamber wall 10. This roller 41 is connected to a pinion 42 coupled to a smaller pinion 43 on a shaft 44 which passes through an opening in a metallic mesh microwave screen 48, and through an aperture in the wall 45, and is supported by bearings 46,47. Also mounted on the shaft 44 is a magnetic driven rotor 50 which is closely associated with a driving magnet 51, but sealed therefrom totally by an intervening wall 52 which acts as a positive fluid seal or enclosure and is in permanent static contact with a cylindrical extension 53 of the chamber wall 10. The magnet 51 is mounted on a drive shaft 55 supported in bearings and secured to a gear 56 forming part of a worm drive coupled to a driving motor 57. Figure 4 illustrates cross-sections through the coupling unit on the lines A-A and B-B in Figure 3.

Thus it will be seen that control movements imparted by the motor 57 cause rotation of the magnet 51 and hence of the driven rotor 50 and the basket drive roller 41. In this way the basket with its contents can be moved continuously or in intervals as required for optimum sterilisation.

The Ethylene Oxide injector illustrated in Figures 5 and 6 is arranged to be totally sealed within the chamber 10 but can be actuated externally. The wall 10 of the chamber has an extension 60 to which is positively attached in a fluid tight manner a cylindrical housing 61 and between the two is located a sleeve 62 having a sharp pointed hollow needle 63 at its inner end arranged when required to perforate a sealed capsule 64 containing liquid Ethylene Oxide. When punctured the liquid contents pass through the needle, impinge upon a baffle plate 59,and are rapidly vapourised into the interior of the chamber. The capsule is forced against the needle by means of an abutment plate 72 which is urged towards the outer end of the capsule by a metal bellows 75 expanded by the pressure of atmospheric air entering the internal void of the bellows via a port 78. The injector unit is permanently sealed off from the atmosphere by the bellows which is closed at its inner end and positively sealed at its outer end to an annulus 77 secured within the cylindrical housing 61. The abutment 72 is provided with a shaft 70 which passes through the void within the bellows and thence through the annulus and the end of the housing 61. The shaft is provided with a manual locking lever which can be pulled outwards and rotated to lock the shaft thus holding the bellows in the contracted position. Evacuation of the main chamber causes the bellows to experience a pressure equal to the difference between atmospheric pressure and chamber pressure. When the shaft is released the pressure differential causes air to enter the internal void of the bellows via the port 78 extending the bellows and driving the abutment plate inwards. For safety reasons this arrangement provides a means of puncturing the Ethylene Oxide canisters which will only operate when the chamber pressure is reduced below atmospheric pressure.

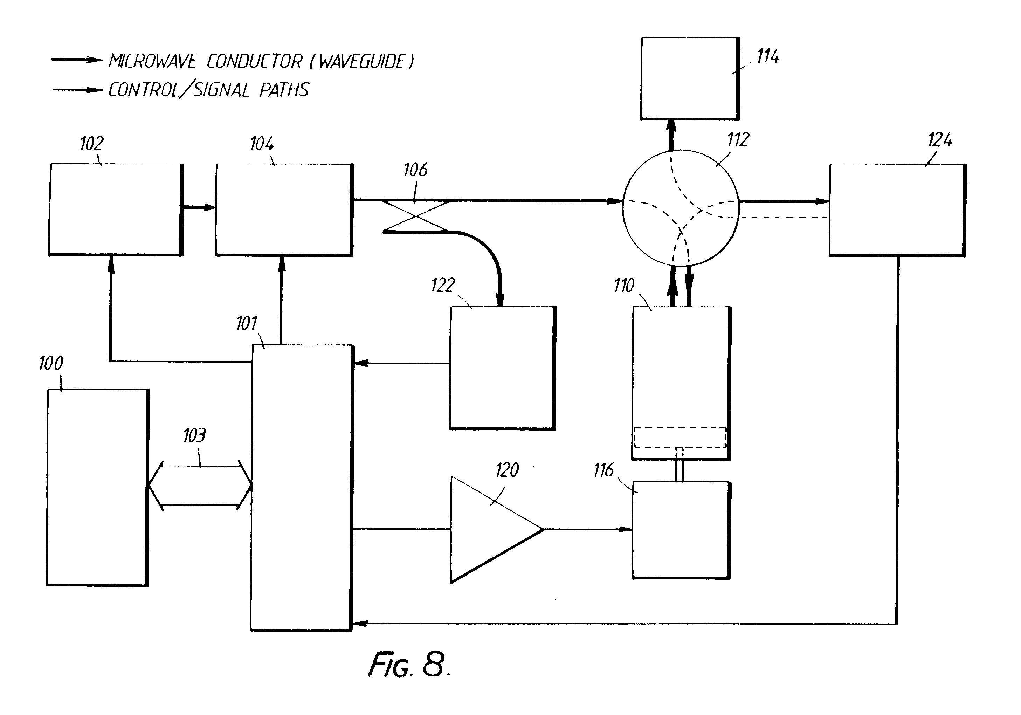

Figure 7 illustrates a microwave molecular rotational spectrometer attached to the chamber 10 for detecting the presence and concentration level of Ethylene Oxide and other gases. Molecular rotational microwave spectrometry is a fundamental analytical technique. Operating on the principle of quantised absorption of electromagnetic radiation, a molecular dipole in the gas phase will exhibit promotion of its rotational state to one of many available energy levels resulting in a highly characteristic narrow bandwith (absorption) spectrum unique to that molecular species; reflecting as it does properties of the entire molecule rather than those of just the nucleus or constituent atoms or groups. Furthermore the magnitude of absorption maxima at resonant frequencies is related to the molecular concentration of the analyte and at low pressures when molecular interactions are at a minimum a linear concentration relationship is established. Absorption "peaks" for many compounds may be detected at frequencies between 10 Gigahertz and 40 Gigahertz. The spectrometer illustrated in Figure 7 is comprised of a rectangular wave guide tube 81 having a Klystron, Gunn diode or other suitable low power monochromatic microwave generator 80 at the lower end and a microwave detector 83 at the upper end. The interior of the tube is connected to and communicates with the interior of the main chamber 10 via two rigid welded branch tubes 82 each equipped with a microwave choke to prevent the escape of microwave energy while permitting the free diffusion of gases and vapours via suitable permeable septa. Within the tube 81 is a thin insulated metal strip electrode positioned centrally along the major axis of the tube. This electrode is connected to an external alternating square wave voltage source such that a potential difference arises between the electrode and the tube during every half of the alternating voltage cycle giving rise to "Stark" splitting of the angular momentum of the molecular dipoles and a corresponding shift in the frequency of the microwave absorption for the particular molecular rotational transition under observation. The microwave detector passes signals to a detector amplifier which incorporates a phase sensitive discriminator operating synchronously with the "Stark" modulated voltage. The detector amplifier output thus reflects microwave energy absorption occurring each half cycle during zero electrode potential. This arrangement improves sensitivity by improving the signal to noise ratio. According to another preferred feature where greater spectrometer sensitivity is required the waveguide tube 81 may be replaced by a microwave resonant cavity of high quality (Q) factor connected to and communicating with the interior of the main chamber as previously described and having a monochromatic microwave generator and a microwave detector at appropriate positions and a central electrode with which to employ the "Stark" effect.

The pressure of the gas sample in the spectrometer is approximately 20 millitorr maintained by a high vacuum pump. Gas molecules diffuse into the spectrometer through permeable septa.

According to yet another preferred feature a microwave rotational spectrometer utilising multiple sequential scanning as a means of signal enhancement which obviates the need for Stark modulation voltages may be used. Figure 8 is a schematic diagram of such a spectrometer control system for use in controlling the process of ethylene oxide sterilisation. In this system a microprocessor unit 100 is arranged to control automatically the operation of the instrument and analyse the data received from the power measuring devices. An interfacing unit 101 includes electronic circuitry to link the microprocessor data bus 103 to the remainder of the spectrometer components. A Gunn Diode oscillator 102, under the control of the microprocessor is arranged to "search" for a specific absorption peak by sweeping a preselected frequency band. The power output from the oscillator is limited by means of a precision attenuator 104. A directional coupling 106 splits the microwave beam from the Gunn Oscillator into two halves so that the power incident upon the gas filled cavity 110 may be continuously monitored by sensing one half of the beam. A waveguide circulator 112 enables the other half of the microwave beam to be fed into/out of the same cavity orifice. This avoids the possibility of interference between the incident and transmitted beams which would adversely affect the accuracy of measurement. A power absorbing load 114 soaks up any microwave power reflected from the measuring devices.

The tunable resonant cavity 110 has its resonant frequency varied electromechanically in order to match it to the frequency of the input radiation. As a result, at resonance the microwave beam will pass through the comparatively small volume of gas very many times, thus greatly increasing the efficiency of the absorption.

The necessary minute changes in cavity geometry are achieved with the desired degree of precision by means of a piezoelectric positioning device 116. This converts changes in an applied voltage into changes in mechanical displacement (up to 100 microns). The device 116 is powered by an amplifier 120 controlled from the microprocessor interfacing unit 101, and there are two power measuring devices 122,124 also associated with the interfacing.

The equipment also includes an infra-red remote pyrometer, illustrated diagrammatically at 90, on the side wall of the chamber 10, which enables temperature readings to be obtained within the chamber without affecting or being affected by the microwave field.

The equipment may also include an internal pressure sensor designed to avoid interference with the microwave field and arranged not to affect the positive sealing of the chamber.

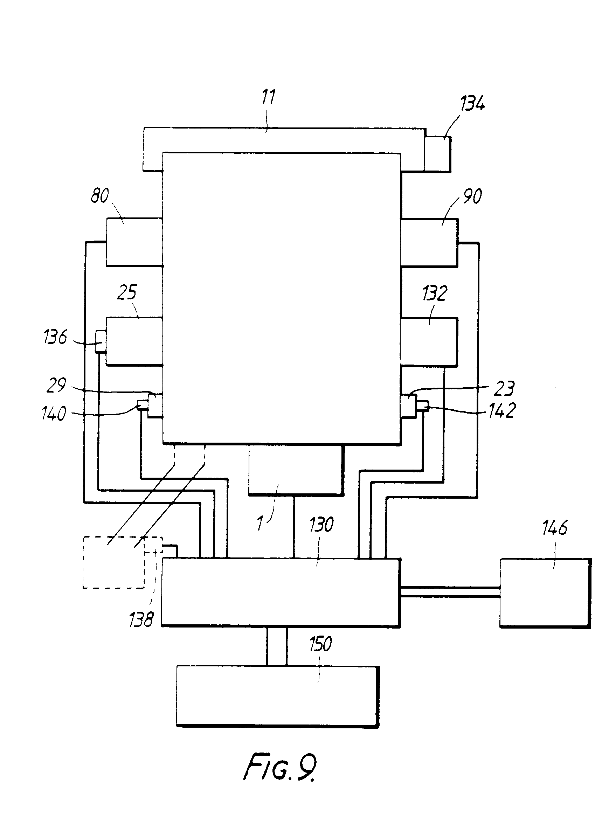

The whole equipment is conveniently controlled by an automatic or semi-automatic control system, as illustrated diagrammatically in Figure 9. One possible layout includes a central microprocessor unit 130 having a number of inputs from sensors associated with the equipment, and output functions which may be fully automatic or may be in the form of indicators for use by an operator. Amongst other possible sensors forming part of the control is the spectrometer 80-85, as illustrated in Figure 7, and in addition the infra-red pyrometer 90, or other temperature sensor, a vacuum gauge or pressure sensor 132 for the interior of the chamber, and a number of safety micro switches, for example switch 134, to indicate that the cover 11 is safely closed and fluid-tight, switch 136 to show that the ethylene oxide injector 25 is also closed, switch 138 to show that the vacuum pump is or is not operating, and switches 140,142 to show that the air vent 29 or the air inlet 23 is open or closed. In addition, there is a multiple function timer 146 providing both a fractional time count for individual steps in the process and an overall time measurement. The microprocessor outputs provide control of solenoid operated valves and other control functions via an interface 150. These automatically controlled function elements include valves or actuators associated with the ethylene oxide injector 25, the air inlet and vent 23,29, the vacuum pump 152, and the microwave generators 32,33,34.

The preferred "duty cycle" for the equipment is as follows:-

(a) Pre-treatment of the objects to be sterilised including partial evacuation of air from the chamber combined with a humidifying step by addition of water vapour to ensure that bacteria spores and vegetative forms are more susceptible to Ethylene Oxide. This may be followed by a flushing out of the chamber with other gases, for example $CO_2$.

(b) The Sterilising Stage.

Ethylene Oxide is admitted under sub-atmospheric partial vacuum conditions and sterilisation continues either for a fixed time or until predetermined parameters are reached as determined by the sensors on the equipment. During this sterilisation the internal pressure in the chamber returns nearly to atmospheric as a result of the injection of Ethylene Oxide.

(c) Flushing.

The Ethylene Oxide is pumped out of the chamber and the pressure reduced to a substantial vacuum. Flushing air is then admitted and the chamber again evacuated and pumped down to a lower vacuum. This pulsing may continue through several cycles.

(d) With the chamber pressure at some predetermined pressure below atmospheric the microwave generator is actuated and allowed to run for a period of time, which may be set by an automatic timer or determined by readings obtained from the sensors particularly the microwave spectrometer which gives an indication of the Ethylene Oxide remnant in the chamber. At the end of the microwave stage the chamber is again pumped out and there may be a further flushing step with clean air. Provided that the parameters are then within the specified ranges, particularly the reading of the spectrometer, the cycle terminates and the cover is automatically unlocked.

## Claims

1. Sterilising apparatus comprising a chamber (10) having an access opening and closure (11) therefor, means (23) for introducing a sterilising gas or vapour into the chamber to contact articles to be sterilised and for extracting the gas or vapour when required, and means (31-34) for generating or radiating high frequency electro-magnetic radiation in or into the chamber, and characterised by means (9) to limit or prevent unwanted escape or discharge of the radiated energy, and means (13) for creating a fluid-tight seal with the closure (11).

2. Apparatus according to Claim 1, characterised in that the closure seal (13) is also provided with means to restrict or prevent escape of electro-magnetic radiation.

3. Apparatus according to Claim 1 or Claim 2, characterised by a movable support (27) for objects to be sterilised within the chamber, and drive means (30) operable from outside the chamber for actuating the support.

4. Apparatus according to Claim 3, characterised in that the drive means (30) includes a drive coupling (50,51,52) between the interior and exterior of the chamber, the coupling being arranged to maintain the positive fluid and radiation seals.

5. Apparatus according to any of the preceding claims, characterised by means to locate within the chamber a sealed container (64) for the sterilising gas or vapour as a gas or liquid and means (63) to discharge the gas or liquid from the container within the chamber, the actuating means being operable from outside the chamber without disturbing the gas or radiation seals.

6. Apparatus according to any of the preceding claims, characterised in that the radiation generator (31-34) is a microwave irradiation system arranged to create or introduce microwave radiation into the chamber through a sealed wall (31) "transparent" to the microwave radiation.

7. Apparatus according to any of the preceding claims, characterised in that the radiation generator (31-34) includes means for varying the level of radiation.

8. Apparatus according to any of the preceding claims, characterised by means for varying or controlling the pressure within the chamber, e.g. above or below atmospheric.

9. A method of sterilising an article in which the article is placed in a sealed chamber (10) forming part of sterilising apparatus according to Claim 1, characterised in that sterilising gas or vapour is introduced into the chamber to contact the article and later withdrawn, high frequency electromagnetic radiation is created or introduced into the chamber to cause irradiation of the article and/or any adsorbed gas or vapour, and hence desorption of the gas or vapour, and the article is then withdrawn from the chamber.

10. A method according to Claim 9, characterised in that the article is introduced into and withdrawn from the chamber through a closure (11) which includes a fluid-tight seal (13) and a radiation seal. (9)

11. A method according to Claim 9 or 10, characterised in that the gas is Ethylene Oxide.

12. A method according to any of Claims 9,10 and 11, characterised in that the sterilising gas, or vapour, is initially contained as a gas or liquid in a sealed capsule or container (64) placed within the chamber (10), and the gas or liquid is released from the container by an actuator (63) operated externally of the chamber.

13. A method according to any of Claims 9,10,11 and 12, characterised in that the temperature within the chamber (10), or the article, is sensed from outside the chamber by an infrared pyrometer (90) or other remote thermal sensor unaffected by the radiation.

14. A method according to any of the preceding Claims 9 to 13, characterised in that the radiation is introduced into the chamber (10) while the sterilising gas or vapour is within the chamber.

15. A method according to any of the preceding Claims 9-14, characterised in that the radiation is created or introduced into the chamber (10) after the sterilising gas or vapour has been withdrawn.

16. A method according to any of the preceding Claims 10-14, characterised in that the article to be sterilised is moved within the chamber (10) during exposure to the gas or vapour and/or the radiation by means of a motivating system (30) actuated externally.

17. Gas desorbing apparatus for use in conjunction with gas sterilising apparatus, comprising a fluid-tight chamber (10) having an access opening and a closure (11) therefor,and means (31-34) for generating or radiating high-frequency electromagnetic radiation in or into the chamber, and characterised by means (9) to limit or prevent unwanted escape or discharge of the radiated energy, fluid tight sealing means (13) for the closure, and means (23) for introducing a cleansing or flushing gas gas or vapour such as air into the chamber and for extracting the said gas or vapour.

18. A method of desorbing gas or vapour absorbed into an article, wherein the article is placed in a chamber (10) (10) having an access opening and a closure (11) therefor, said chamber (10) having means (9) for limiting or preventing unwanted escape or discharge of the radiated energy and fluid tight sealing means (13) for the closure (11), high frequency electromagnetic radiation is created in or introduced into the chamber to cause desorption of the gas or vapour and the article is then removed from the chamber.

19. A method according to Claim 18, characterised in that a flushing or cleansing gas or vapour such as air is passed through the chamber (10) before, during or after the irradiation process.

20. Apparatus according to any of Claims 1 to 8, or 17, characterised by an automatic or semi-automatic system (130) for controlling the cycle, comprising one or more sensors (80,90 et al) for detecting selected parameters of the operating conditions such as chamber pressure, or temperature, radiation level, concentration of gas or vapour, and physical state of actuating elements, together with a programmed controller, and automatic control function elements actuated by the controller for performing selected steps in the cycle.

21. Apparatus according to any of Claims 1 to 8, or 17, or 20, characterised by means for filtering or separating the gas or vapour within the chamber, e.g. an adsorption agent including a high molecular weight polymeric material.

22. Apparatus according to Claim 21, characterised by means for subsequently reclaiming materials absorbed by the filtering or separating means.

23. A method of sterilising an article in which the article is introduced through the entrance opening of a chamber (10) forming part of sterilising apparatus according to Claim 1, the opening is closed and a gastight and radiation resistance seal created, sterilising gas or vapour is introduced into the chamber to make contact with the article, and while the gas or vapour is in contact with the article high frequency electro-magnetic radiation is created in or introduced into the chamber.

24. Apparatus for sensing or measuring a gas vapour or constituent, comprising a chamber (110) into which the gas vapour or constituent is introduced, means (102,104) for generating or introducing microwave radiation in the chamber and means (116,120) for tuning the resonant cavity afforded by the chamber, characterised in that the tuning means comprises a piezoelectric positioning element connected to a movable member or wall in the chamber (110).

25. Apparatus for sensing or measuring a gas vapour or constituent, comprising a chamber (110) into which the gas vapour or constituent is introduced, means (102,104) for generating or introducing microwave radiation in the chamber and means (116,120) for tuning the resonant cavity afforded by the chamber, characterised in that multiple sequential scanning modulation is applied to at least one of the resonant frequency of the resonant cavity, and the frequency of the microwave radiation.

26. Apparatus according to Claim 24 or Claim 25, characterised in that the resonant cavity (110) is tuned automatically in response to changes in the sensed energy level (124) in the chamber.

27. Gas desorbing apparatus comprising a chamber (10) having an access opening and a closure (11) therefor, and means (31,34) for generating or radiating high frequency electromagnetic radiation in or into the chamber and characterised by means (9) to prevent unwanted escape or discharge of the radiated energy, means (13) for creating a fluid tight seal with the closure (11), and means for introducing a cleansing or flushing gas or vapour into the chamber and extracting said gas or vapour..

28. Sterilising apparatus comprising a chamber (10) having an access opening and closure (11) therefor, means (13) for creating a fluid tight seal with the closure (11), and means (23) for introducing a sterilising gas or vapour into the chamber to contact articles to be sterilised and for extracting the gas or vapour when required, characterised in that said apparatus has associated therewith means (24) for sensing or measuring a gas, vapour or constituent, said means including a sensing chamber (110) into which the gas, vapour or constituent, is introduced, means (102, 104) for generating or introducing microwave radiation in the chamber and means (116,120) for tuning the resonant cavity afforded by the chamber.

29. A gas desorption system including gas desorbing apparatus according to Claim 17 or Claim 27, in conjunction with gas sensing or measuring apparatus according to any one of Claims 24 to 26.

30. A gas sterilising system including:

(a) gas sterilising apparatus having a sterilising chamber and means for filtering or separating gas or vapour within the sterilising chamber, in conjunction with
(b) a gas desorbing apparatus according to Claim 17.

31. A gas sterilising system according to Claim 30, wherein said filtering or separating means comprises an adsorption agent including a high molecular weight polymeric material.

**Revendications**

1. Appareil de stérilisation comportant une chambre (10) pourvue d'un orifice d'accès et d'un élément de fermeture (11) pour ce dernier, des moyens (23) pour introduire un gaz ou une vapeur de stérilisation dans la chambre au contact d'objets destinés à être stérilisés et pour, lorsque cela est nécessaire, extraire le gaz ou la vapeur, et des moyens (31-34) pour générer ou émettre un rayonnement électromagnétique à haute fréquence dans la chambre, et caractérisé par des moyens (9) pour limiter ou empêcher une fuite ou une libération non voulue de l'énergie rayonnée, et par des moyens (13) pour créer avec l'élément de fermeture (11) un joint hermétique étanche à un fluide.

2. Appareil selon la revendication 1, caractérisé en ce que le joint d'étanchéité (13) de l'élément de fermeture est également pourvu de moyens pour limiter ou empêcher une fuite d'un rayonnement électromagnétique.

3. Appareil selon la revendication 1 ou la revendication 2, caractérisé par un support mobile (27) destinés à des objets devant être stérilisés à l'intérieur de la chambre, et des moyens d'entraînement (30) aptes à être commandés de l'extérieur de la chambre pour actionner le support.

4. Appareil selon la revendication 3, caractérisé en ce que les moyens d'actionnement (30) comportent un accouplement d'entraînement (50, 51, 52) entre l'intérieur et l'extérieur de la chambre, l'accouplement étant conçu pour maintenir les joints d'étanchéité positifs à un fluide et à un rayonnement.

5. Appareil selon l'une quelconque des revendications précédentes, caractérisé par des moyens pour placer à l'intérieur de la chambre un récipient fermé hermétiquement (64) destiné à contenir le gaz ou la vapeur de stérilisation, sous la forme d'un gaz ou d'un liquide, et des moyens (63) pour évacuer le gaz ou le liquide hors du récipient à l'intérieur de la chambre, les moyens d'actionnement pouvant être commandés de l'extérieur de la chambre sans détériorer les joints d'étanchéité à un gaz ou à un rayonnement.

6. Appareil selon l'une quelconque des revendications précédentes, caractérisé en ce que le générateur de rayonnement (31-34) est un système d'irradiation d'ondes ultracourtes conçu pour générer ou introduire un rayonnement d'ondes ultracourtes dans la chambre à travers une paroi fermée hermétiquement (31) "transparente" au rayonnement d'ondes ultracourtes.

7. Appareil selon l'une quelconque des revendications précédentes, caractérisé en ce que le générateur de rayonnement (31-34) comporte des moyens pour faire varier le niveau de rayonnement.

8. Appareil selon l'une quelconque des revendications précédentes, caractérisé par des moyens pour faire varier ou pour contrôle la pression à l'intérieur de la chambre, par exemple, au-dessus ou au-dessous de la pression atmosphérique.

9. Procédé pour stériliser un objet, selon lequel l'objet est placé dans une chambre fermée hermétiquement (10) faisant partie d'un appareil de stérilisation selon la revendication 1, caractérisé en ce qu'il consiste à introduire, pour l'extraire ultérieurement, un gaz ou une vapeur de stérilisation dans la chambre au contact de l'objet, à générer ou à introduire un rayonnement électromagnétique à haute fréquence dans la chambre pour provoquer une irradiation de l'objet et/ou d'un gaz ou d'une vapeur quelconque adsorbé(e), et, par conséquent, une désorption du gaz ou de la vapeur, et à extraire ensuite l'objet de la chambre.

10. Procédé selon la revendication 9, caractérisé en ce que l'objet est introduit dans la chambre et extrait de celle-ci par l'intermédiaire d'un élément de fermeture (11) comportant un joint hermétique (13) étanche à un fluide et un joint (9) d'étanchéité à un rayonnement.

11. Procédé selon la revendication 9 ou 10, caractérisé en ce que le gaz est de l'Oxyde d'Ethylène.

12. Procédé selon l'une quelconque des revendications 9, 10 et 11, caractérisé en ce que le gaz ou la vapeur de stérilisation est initialement contenu(e) sous la forme d'un gaz ou d'un liquide dans une capsule ou un récipient fermé(e) hermétiquement (64) qui est placé(e) à l'intérieur de la chambre (10), le gaz ou le liquide étant libéré du récipient au moyen d'un organe d'actionnement (63) commandé extérieurement à la chambre.

13. Procédé selon l'une quelconque des revendications 9, 10, 11 et 12, caractérisé en ce que la température à l'intérieur de la chambre (10), ou celle de l'objet, est détectée de l'extérieur de la chambre à l'aide d'un pyromètre à infrarouge (90) ou d'un autre capteur thermique à distance insensible au rayonnement.

14. Procédé selon l'une quelconque des revendications 9 à 13 précédentes, caractérisé en ce que le rayonnement est introduit dans la chambre (10) pendant que le gaz ou la vapeur de stérilisation est à l'intérieur de celle-ci.

15. Procédé selon l'une quelconque des revendications 9 à 14 précédentes, caractérisé en ce que le rayonnement est engendré ou introduit dans la chambre (10) après extraction du gaz ou de la vapeur de stérilisation.

16. Procédé selon l'une quelconque des revendications 10 à 14 précédentes, caractérisé en ce que l'objet destiné à être stérilisé est déplacé à l'intérieur de la chambre (10) pendant son exposition au gaz ou à la vapeur et/ou au rayonnement, au moyen d'un système d'entraînement (30) actionné extérieurement.

17. Appareil de désorption d'un gaz, destiné à être utilisé en association avec un appareil de stérilisation au gaz, comportant une chambre (10) étanche à un fluide pourvue d'un orifice d'accès et d'un élément de fermeture (11) pour ce dernier, et des moyens (31-34) pour générer ou émettre un rayonnement électromagnétique à haute fréquence dans la chambre, et caractérisé par des moyens (9) pour limiter ou empêcher une fuite ou une libération non voulue de l'énergie rayonnée, des moyens (13) formant un joint hermétique étanche à un fluide pour l'élément de fermeture, et des moyens (23) pour introduire dans la chambre un gaz ou une vapeur de nettoyage ou de rinçage, comme de l'air, et pour extraire ledit gaz ou ladite vapeur.

18. Procédé de désorption d'un gaz ou d'une vapeur adsorbé(e) dans un objet, consistant à placer l'objet dans une chambre (10) pourvue d'un orifice d'accès et d'un élément de fermeture (11) pour ce dernier, ladite chambre (10) possédant des moyens (9) pour limiter ou empêcher une fuite ou une libération non voulue de l'énergie rayonnée et des moyens (13) forment un joint hermétique étanche à un fluide pour l'élément de fermeture (11), à générer ou à introduire dans la chambre un rayonnement électromagnétique à haute fréquence pour provoquer une désorption du gaz ou de la vapeur, et à extraire ensuite l'objet de la chambre.

19. Procédé selon la revendication 18, caractérisé en ce que l'on fait passer un gaz ou une vapeur de nettoyage ou de rinçage, comme de l'air, à travers la chambre (10) avant, pendant ou après le processus d'irradiation.

20. Appareil selon l'une quelconque des revendications 1 à 8, ou 17, caractérisé par un système automatique ou semi-automatique (130) pour commander le cycle, comportant un ou plusieurs détecteurs (80, 90 etc.) pour détecter des paramètres sélectionnés des conditions de fonctionnement, telles qu'une pression de la chambre, ou une température, un niveau de radiation, une concentration de gaz ou de vapeur, et un état physique d'éléments d'actionnement, conjointement avec un organe de commande programmé, et des éléments assurant des fonctions de commande automatique actionnés par l'organe de commande pour exécuter des étapes sélectionnées du cycle.

21. Appareil selon l'une quelconque des revendications 1 à 8, ou 17, ou 20, caractérisé par des moyens pour filtrer ou séparer le gaz ou la vapeur à l'intérieur de la chambre, par exemple, un agent d'adsorption qui contient une substance polymère ayant masse moléculaire élevée.

22. Appareil selon la revendication 21, caractérisé par des moyens pour récupérer ensuite des substances absorbées par les moyens de filtration ou de séparation.

23. Procédé pour stériliser un objet consistant à introduire l'objet par l'orifice d'entrée d'une chambre (10) qui fait partie d'un appareil de stérilisation selon la revendication 1, à fermer l'orifice et à créer un joint hermétique étanche à un gaz et résistant à un rayonnement, à introduire un gaz ou une vapeur de stérilisation dans la chambre pour l'amener en contact avec l'objet, et, pendant que le gaz ou la vapeur est en contact avec l'objet, à générer ou à introduire dans la chambre un rayonnement électromagnétique à haute fréquence.

24. Appareil pour détecter ou mesurer un gaz, une vapeur ou un constituant, comportant une chambre (110) dans laquelle le gaz, le vapeur ou le constituant est introduit(e), des moyens (102, 104) pour générer ou introduire un rayonnement d'ondes ultracourtes dans la chambre et des moyens (116, 120) pour accorder la cavité résonante définie par la chambre, caractérisé en ce que les moyens d'accord comportent un élément de positionnement piézo-électrique relié à un organe ou à une paroi mobile dans la chambre (110).

25. Appareil pour détecter ou mesurer un gaz, une vapeur ou un constituant, comportant une chambre (110) dans laquelle le gaz, la vapeur ou le constituant est introduit(e), des moyens (102, 104) pour générer ou introduire un rayonnement d'ondes ultracourtes dans la chambre et des moyens (116, 120) pour accorder la cavité résonante définie par la chambre, caractérisé en ce qu'une modulation à balayage séquentiel multiple est appliquée à l'une au moins de la fréquence de résonance de la cavité résonante et de la fréquence du rayonnement d'ondes ultracourtes.

26. Appareil selon la revendication 24 ou la revendication 25, caractérisé en ce que la cavité résonante (110) est accordée automatiquement en réponse à des variations du niveau d'énergie détecté (124) dans la chambre.

27. Appareil de désorption d'un gaz, comportant une chambre (10) pourvue d'un orifice d'accès et d'un élément de fermeture (11) pour ce dernier, et des moyens (31, 34) pour produire ou émettre un rayonnement électromagnétique à haute fréquence dans la chambre et caractérisé par des moyens (9) pour empêcher une fuite ou une libération non voulue de l'énergie rayonnée, des moyens (13) pour créer avec l'élément de fermeture (11) un joint hermétique étanche à un fluide, et des moyens pour introduire un gaz ou une vapeur de nettoyage ou de rinçage dans la chambre et pour extraire ledit gaz ou ladite vapeur.

28. Appareil de stérilisation comportant une chambre (10) pourvue d'un orifice d'accès et d'un élément de fermeture (11) pour ce dernier, des moyens (13) pour créer avec l'élément de fermeture (11) un joint hermétique étanche à un fluide, et des moyens (23) pour introduire un gaz ou une vapeur de stérilisation dans la chambre au contact d'objets destinés à être stérilisés et pour, lorsque cela est nécessaire, extraire le gaz ou la vapeur, caractérisé en ce que ledit appareil possède, associés à lui, des moyens (24) pour détecter ou mesurer un gaz, une vapeur ou un constituant, lesdits moyens comportant une chambre de détection (110) dans laquelle le gaz, la vapeur ou le constituant est introduit(3), des moyens (102, 104) pour générer ou introduire un rayonnement d'ondes ultracourtes dans la chambre et des moyens (116, 120) pour accorder la cavité résonante définie par la chambre.

29. Système de désorption de gaz, comportant un appareil de désorption d'un gaz, selon la revendication 17 ou la revendication 27, en association avec un appareil pour détecter ou mesurer un gaz selon l'une quelconque des revendications 24 à 26.

30. Système de stérilisation au gaz, comprenant :
    (a) un appareil de stérilisation au gaz, comportant une chambre de stérilisation et des moyens pour filtrer ou séparer un gaz ou une vapeur à l'intérieur de la chambre de stérilisation, en association avec
    (b) un appareil de désorption d'un gaz, selon la revendication 17.

31. Système de stérilisation au gaz, selon la revendication 30, dans lequel lesdits moyens de filtration ou de séparation comportent un agent d'adsorption qui contient une substance polymère ayant une masse moléculaire élevée.

**Patentansprüche**

1. Sterilisationsvorrichtung, enthaltend eine Kammer (10) mit einer Zutrittsöffnung und einem Verschluß (11) hiertur, mit Mitteln (23) zum Einführen von Stevilisiergas oder -dampf in die Kammer zur Berührung mit zu sterilisierenden Gegenständen und zum Abziehen des Gases oder Dampfes, wenn dies erwünscht ist, und mit Mitteln (31 bis 34) zum Erzeugen oder Bestrahlen elektromagnetischer Strahlung hoher Frequenz in der oder in die Kammer, gekennzeichnet durch Mittel (9) zum Begrenzen oder Verhindern von unerwünschtem Austreten oder Entladen der Strahlungsenergie und Mittel (13) zum Erzeugen einer fluiddichten Abdichtung mit dem Verschluß (11).

2. Vorrichtung nach Anspruch 1, dadurch gekennzeichnet, daß die Verschlußdichtung (13) auch mit Mitteln zum Begrenzen oder Verhindern des Austretens elektromagnetischer Strahlung versehen ist.

3. Vorrichtung nach Anspruch 1 oder 2, gekennzeichnet durch einen bewegbaren Träger (27) für zu sterilisierende Gegenstände innerhalb der Kammer und außerhalb der Kammer operierende Antriebsmittel (30) zum Betätigen des Trägers (27).

4. Vorrichtung nach Anspruch 3, dadurch gekennzeichnet, daß die Antriebsmittel (30) eine Antriebskupplung (50, 51, 52) zwischen der Innen- und Außenseite der Kammer beinhalten, wobei die Kupplung so ausgebildet ist, daß die Fluid- und Strahlungsabdichtungen bestehen bleiben.

5. Vorrichtung nach einem der vorhergehenden Ansprüche, gekennzeichnet durch Mittel zum Fixieren eines dichten Behälters (64) innerhalb der Kammer für Sterilisiergas oder -dampf, wie Gas oder Flüssigkeit, und Mittel (63) zum Ablassen von Gas oder Flüssigkeit aus dem Behälter in die Kammer, wobei die Betätigungsmittel von der Außenseite der Kammer bedienbar sind, ohne die Dichtungen für Gas oder Strahlung Zu beeinträchtigen.

6. Vorrichtung nach einem der vorhergehenden Ansprüche, dadurch gekennzeichnet, daß der Strahlungsgenerator (31 bis 34) ein Mikrowellen-Strahlungssystem ist und zum Erzeugen oder Einstrahlen von Mikrowellen in die Kammer durch eine abgedichtete Wand (31), die "transparent" für Mikrowellen ist, dient.

7. Vorrichtung nach einem der vorhergehenden Ansprüche, dadurch gekennzeichnet, daß der Strahlungsgenerator (31 bis 34) Mittel zum Verändern des Strahlungspegels aufweist.

8. Vorrichtung nach einem der vorhergehenden Ansprüche, gekennzeichnet durch Mittel zum Verändern oder Steuern des Druckes in der Kammer, beispielsweise oberhalb oder unterhalb des atmosphärischen Druckes.

9. Verfahren zum Sterilisieren eines Gegenstandes, bei dem der Gegenstand sich im Innern einer abgedichteten Kammer (10) befindet, die Bestandteil einer Sterilisationsvorrichtung gemäß Anspruch 1 ist, dadurch gekennzeichnet, daß Sterilisierungsgas oder -dampf in die Kammer zwecks Berührung mit dem Gegenstand eingeführt und später wieder entfernt wird, daß hochfrequente elektromagnetische Strahlung erzeugt oder in die Kammer eingestrahlt wird, um eine Bestrahlung des Gegenstands und/oder ein adsorbiertes Gas oder Dampf und eine Desorbierung des Gases oder des Dampfes zu bewirken, wonach der Gegenstand aus der Kammer entfernt wird.

10. Verfahren nach Anspruch 9, dadurch gekennzeichnet, daß der Gegenstand in die Kammer eingeführt und aus dieser entfernt wird durch eine Öffnung, welche eine fluiddichte Abdichtung (13) und eine Strahlungsdichtung (9) einschließt.

11. Verfahren nach Anspruch 9 oder 10, dadurch gekennzeichnet, daß das Gas Ethylenoxid ist.

12. Verfahren nach einem der Ansprüche 9, 10 oder 11, dadurch gekennzeichnet, daß das Gas oder der Dampf zum Sterilisieren anfänglich als Gas oder Flüssigkeit in einer geschlossenen Kapsel oder einem Behälter (61) enthalten ist, die bzw. der sich in der Kammer (10) befindet, und daß das Gas oder die Flüssigkeit mit Hilfe eines Organs (63), welches von außerhalb der Kammer steuerbar ist, aus dem Behälter abgelassen wird.

13. Verfahren nach einem der Ansprüche 9, 10, 11 oder 12, dadurch gekennzeichnet, daß die Temperatur in der Kammer (10) oder des Gegenstandes von außerhalb der Kammer erfaßt wird entweder durch ein Infrarot-Pyrometer (90) oder einen anderen fernsteuerbaren Thermosensor, der durch die Strahlung nicht beeinflußt wird.

14. Verfahren nach einem der Ansprüche 9 bis 13, dadurch gekennzeichnet, daß die Strahlung in die Kammer (10) eingeleitet wird, während sich Sterilisierungsgas oder -dampf in der Kammer befindet.

15. Verfahren nach einem der Ansprüche 9 bis 14, dadurch gekennzeichnet, daß die Strahlung in der Kammer (10) hervorgerufen oder eingeführt wird, nachdem das Sterilisationsgas oder der Dampf entfernt wurde.

16. Verfahren nach einem der Ansprüche 10 bis 14, dadurch gekennzeichnet, daß der zu sterilisierende Gegenstand in die Kammer (10) eingebracht wird, während das Gas oder der Dampf und/oder die Strahlung durch Mittel eines Betätigungssystems (30) , welches von außen steuerbar ist, zurückgehalten werden.

17. Gasdesorbierungsvorrichtung zur Benutzung in Verbindung mit einer Gassterilisationsvorrichtung, enthaltend eine strömungsdichte Kammer (10) mit einer Zuführungsöffnung und einem zugehörigen Verschluß (11) und Mitteln (31 bis 34) zum Erzeugen oder Abstrahlen hochfrequenter elektromagnetischer Strahlung in der Kammer oder in die Kammer, gekennzeichnet durch Mittel (9) zum Begrenzen oder Verhindern unerwünschter Abstrahlung oder Entladung der Strahlungsenergie, durch fluiddichte Abdichtungsmittel (13) für den Verschluß und durch Mittel (23) zur Einführung eines Reinigungs- oder Spülungsgases oder -dampfes, wie beispielsweise Luft, in die Kammer und zum Austreiben des Gases bzw. des Dampfes.

18. Verfahren zum Desorbieren von Gas oder Dampf, welches bzw. welcher von einem Gegenstand absoiert wurde, wobei der Gegenstand in einer Kammer (10) mit einer Einführungsöffnung und einem zugehörigen Verschluß (11) enthalten ist, bei dem die Kammer (10) mit Mitteln (9) zum Begrenzen bzw. Verhindern unerwünschter Anströmung oder Entladung der Strahlungsenergie und fluiddichten Abdichtmitteln für den Verschluß (11) versehen ist und die hochfrequente elektromagnetische Strahlung in der Kammer erzeugt wird oder in die Kammer eingeleitet wird, um das Gas oder den Dampf auszutreiben und der Gegenstand dann aus der Kammer entfernt wird.

19. Verfahren nach Anspruch 18, dadurch gekennzeichnet, daß ein Reinigungs- oder Spülungsgas oder -dampf, wie beispielsweise Luft, vor, während oder nach dem Strahlungsprozeß durch die Kammer (10) geleitet wird.

20. Vorrichtung nach einem der Ansprüche 1 bis 8 oder 17, gekennzeichnet durch ein automatisches oder halbautomatisches System (130) zum Steuern der Zyklen, enthaltend einen oder mehrere Sensoren (80, 90 u.a.) zum Erfassen selektiver Parameter der Verfahrensbedingungen, wie zum Beispiel Kammerdruck, Temperatur, Strahlungshöhe, Konzentration von Gas oder Dampf und den Zustand der Betätigungselemente in Verbindung mit einem programmierten Steuergerät und automatischen Funktionssteuerelementen, betätigt durch das Steuergerät zur Ausführung ausgewählter Schritte in dem Zyklus,

21. Vorrichtung nach einem der Ansprüche 1 bis 8, oder 17 oder 20, gekennzeichnet durch Mittel zum Filtern oder Trennen des Gases oder Dampfes in der Kammer, wie beispielsweise ein Absorbtionsmittel, enthaltend ein polymeres Material mit hohem Molekulargewicht.

**22.** Vorrichtung nach Anspruch 21, gekennzeichnet durch Mittel zum aufeinanderfolgenden Zurückgewinnen von Materialien, die durch die Filter- oder Trennmittel absorbiert wurden.

**23.** Verfahren zum Sterilisieren eines Gegenstandes, der durch die Eingangsöffnung einer Kammer (10) in diese eingeführt wurde, die Bestandteil einer Sterilisationsvorrichtung gemäß Anspruch 1 ist, wobei die Öffnung geschlossen ist und eine gasdichte und gegen Bestrahlung widerstandsfähige Dichtung gegeben ist und Gas oder Dampf in die Kammer eingeführt ist, um mit dem Gegenstand in Berührung zu kommen und wobei während dieser Berührung hochfrequente elektromagnetische Strahlung in der Kammer erzeugt oder in diese eingeführt wird.

**24.** Vorrichtung zum Erfassen oder Messen von Gasdampf oder einem Bestandteil hiervon, enthaltend eine Kammer (110), in die der Gasdampf oder Bestandteil eingeführt ist, Mittel (102, 104) zum Erzeugen oder Einleiten einer Mikrowellenstrahlung in die Kammer und Mittel (116, 120) zum Abstimmen der durch die Kammer gegebenen Raumresonanz, dadurch gekennzeichnet, daß die Abstimmittel ein piezoelektrisches Stellglied aufweisen, welches mit einem bewegbaren Element oder einer bewegbaren Wand in der Kammer (110) verbunden ist.

**25.** Vorrichtung zum Erfassen oder Messen von Gasdampf oder einem Bestandteil hiervon, enthaltend eine Kammer (110), in die der Gasdampf oder Bestandteil eingeführt ist, Mittel (102, 104) zum Erzeugen oder Einleiten einer Mikrowellenstrahlung in die Kammer und Mittel (116, 120) zum Abstimmen der durch die Kammer gegebenen Raumresonanz, dadurch gekennzeichnet, daß eine Vielfachabtastmodulation wenigstens an die Resonanzfrequenzen der Raumresonanz und die Frequenz der Mikrowellenstrahlung angelegt ist.

**26.** Vorrichtung nach Anspruch 24 oder 25, dadurch gekennzeichnet, daß die Raumresonanz selbsttätig in Abhängigkeit von den Änderungen des erfaßten Energiepegels (124) in der Kammer abstimmbar ist.

**27.** Vorrichtung zum Desorbieren von Gas, enthaltend eine Kammer (10) mit einer Einführungsöffnung und einem Verschluß (11) hierfür, mit Mitteln (31, 34) zum Erzeugen oder Ausstrahlen hochfrequenter elektromagnetischer Strahlung in der Kammer oder in die Kammer, gekennzeichnet durch Mittel (9) zum Begrenzen oder Verhindern unerwünschter Abstrahlung oder Entladung der Strahlungsenergie, durch fluiddichte Abdichtungsmittel (13) für den Verschluß und durch Mittel (23) zur Einführung eines Reinigungs- oder Spülungsgases oder -dampfes, wie beispielsweise Luft, in die Kammer und zum Abziehen des Gases bzw. des Dampfes.

**28.** Sterilisationsvorrichtung, enthaltend eine Kammer (10) mit einer Einführungsöffnung und einem Verschluß (11) hierfür, mit Mitteln (13) zur Bildung einer fluiddichten Abdichtung mit dem Verschluß (11) und Mitteln (23) zum Einleiten von Sterilisiergas oder -dampf in die Kammer zwecks Berührung mit den zu sterilisierenden Gegenständen und zum Entfernen des Gases oder Dampfes, wenn dies erwünscht ist, dadurch gekennzeichnet, daß die Vorrichtung zugehörige Mittel (24) zum Erfassen oder Messen von Gas, Dampf oder Bestandteile hiervon aufweist, wobei die Mittel eine Sensorkammer (110) beinhalten, in die Gas, Dampf oder Bestandteile eingeleitet sind, daß ferner Mittel (102, 104) zum Erzeugen oder Einführen von Mikrowellenstrahlung in die Kammer und Mittel (116, 120) zum Einstellen der durch die Kammer gegebenen Raumresonanz vorgesehen sind.

**29.** System zum Desorbieren von Gas, enthaltend eine Gasdesorbierungsvorrichtung gemäß Anspruch 17 oder Anspruch 21 in Verbindung mit Einrichtungen zum Erfassen oder Messen von Gas gemäß einem der Ansprüche 24 bis 26.

**30.** Gassterilisationssystem, enthaltend
a) eine Gassterilisationsvorrichtung mit einer Sterilisierkammer und Mitteln zum Filtern oder Trennen von Gas oder Dampf in der Sterilisierkammer in Verbindung mit
b) einer Gasdesorbierungsvorrichtung gemäß Anspruch 17.

**31.** Gassterilisationssystem gemäß Anspruch 30, bei dem die Mittel zum Filtern oder Trennen ein Adsorbtionsmittel mit einem polymeren Material mit hohem Molekulargewicht einschließen.

FIG.1.

FIG.2.

FIG. 3.

FIG. 4.

FIG.5.

FIG.6.

FIG.7.

MICROWAVE CONDUCTOR (WAVEGUIDE)

CONTROL/SIGNAL PATHS

FIG. 8.

EP 0 201 553 B1

FIG. 9.